# EUROPÄISCHE PATENTSCHRIFT

(11) **EP 1 998 692 B1**
(45) Veröffentlichungstag und Bekanntmachung des Hinweises auf die Patenterteilung: **13.10.2010**
(21) Anmeldenummer: 07727235.9
(22) Anmeldetag: 22.03.2007
(51) Int. Cl.: A61B 17/34

(54) **CHIRURGISCHES DICHTELEMENT, CHIRURGISCHE DICHTUNG UND CHIRURGISCHES ABDICHTUNGSSYSTEM**
SURGICAL SEALING ELEMENT, SURGICAL SEAL, AND SURGICAL SEALING SYSTEM
ELEMENT CHIRGURGICAL D'ETANCHEITE, JOINT CHIRURGICAL ET SYSTEME CHIRURGICAL D'ETANCHEITE

(30) Priorität: 27.03.2006 DE 102006015690
(43) Veröffentlichungstag der Anmeldung: 10.12.2008
(73) Patentinhaber: Aesculap AG, 78532 Tuttlingen (DE)
(72) Erfinder: SCHWEITZER, Tom, 78532 Tuttlingen (DE); MAYENBERGER, Rupert, 78239 Rielasingen (DE)
(74) Vertreter: Hoeger, Stellrecht & Partner Patentanwälte
(86) Internationale Anmeldenummer: PCT/EP2007/052760
(87) Internationale Veröffentlichungsnummer: WO 2007/110371

(56) Entgegenhaltungen:
- WO-A-98/50093
- US-A1- 2005 288 634

## Beschreibung

Die vorliegende Erfindung betrifft ein chirurgisches Dichtelement, insbesondere für einen Trokar und/oder zum Abdichten von Schäften langgestreckter chirurgischer Instrumente beim Einführen in einen menschlichen oder tierischen Körper, wobei das Dichtelement eine Längsachse definiert, mit einer im Durchmesser veränderlichen und quer oder im Wesentlichen quer zur Längsachse orientierten Öffnung versehen ist, durch die ein Instrument eingeführt werden kann, und eine ringförmig geschlossene, flexible Wand umfasst, wobei die Wand einen ersten und einen zweiten, jeweils in sich geschlossenen Rand aufweist und wobei der erste Rand die Öffnung begrenzt.

Ein solches Element ist aus US 2005/0288634 bekannt.

Ferner betrifft die vorliegende Erfindung eine chirurgische Dichtung, insbesondere für einen Trokar und/oder zum Abdichten von Schäften langgestreckter chirurgischer Instrumente beim Einführen in einen menschlichen oder tierischen Körper, wobei die Dichtung ein erstes Dichtelement umfasst, welches eine Längsachse definiert, mit einer im Durchmesser veränderlichen und quer oder im Wesentlichen quer zur Längsachse orientierten Öffnung versehen ist, durch die ein Instrument eingeführt werden kann, und eine erste ringförmig geschlossene, flexible Wand umfasst, wobei die erste Wand einen ersten und einen zweiten, jeweils in sich geschlossenen Rand aufweist und wobei der erste Rand die Öffnung begrenzt.

Des Weiteren betrifft die vorliegende Erfindung ein chirurgisches Abdichtungssystem zum Einführen chirurgischer Instrumente in einen menschlichen oder tierischen Körper, vorzugsweise längs einer vom Abdichtungssystem definierten Längsachse, umfassend einen eine Einführöffnung aufweisenden Trokar und ein die Einführöffnung mindestens teilweise verschließendes, eine Öffnung aufweisendes chirurgisches Dichtelement zum Abdichten der Einführöffnung beim Einführen eines Instruments in die Öffnung.

Bei endoskopischen Operationen am menschlichen oder tierischen Körper wird häufig zur Erweiterung des Operationssitus ein physiologisch unbedenkliches Gas unter leichtem Überdruck in den Körper gefüllt. Damit der Körper in einem dann etwas aufgeblähten Zustand gehalten werden kann, ohne übermäßig viel Gas nachfüllen zu müssen, werden beispielsweise chirurgische Abdichtungssysteme der eingangs beschriebenen Art verwendet. Diese sind üblicherweise in Form eines Trokars ausgebildet, der zwei Dichtungen aufweist. Eine der Dichtungen übernimmt die Abdichtung des Zugangs, also einer Einführöffnung des Trokars, solange kein Instrument eingeführt ist. Eine weitere Dichtung dient dem Zweck, einen Schaft des eingeführten Instruments an seinem Umfang vollständig abzudichten, um einen Gasverlust durch die eingeführten Instrumente zu verhindern.

Da für endoskopische Eingriffe Instrumente mit unterschiedlichen Schaftdurchmessern verwendet werden, sind üblicherweise Dichtungen mit unterschiedlichen Innendurchmessern im Einsatz. Um Instrumentenschäfte mit Durchmessern in einem Bereich von 5 mm bis 15 mm sicher abzudichten, werden zum Beispiel verschiedene Dichtungen mit Innendurchmessern in einem Bereich von 3mm bis 12 mm verwendet. Insbesondere kommen Dichtungen mit Innendurchmessern von 3, 5, 10 und 12 mm Innendurchmesser im ungedehnten Zustand zum Einsatz. Daher ist es in vielen Fällen erforderlich, mehrere Zugänge mit an unterschiedliche Instrumentenschaftdurchmesser angepasste Dichtungen aufweisenden Trokaren vorzusehen oder auch während eines chirurgischen Eingriffs die Dichtungen auszutauschen.

Es ist daher Aufgabe der vorliegenden Erfindung, ein chirurgisches Dichtelement, eine chirurgische Dichtung sowie ein chirurgisches Abdichtungssystem der eingangs beschriebenen Art so zu verbessern, dass unabhängig von ihrem Instrumentenschaftdurchmesser alle gängigen Instrumente zuverlässig abgedichtet werden können.

Diese Aufgabe wird bei einem chirurgischen Dichtelement der eingangs beschriebenen Art erfindungsgemäß dadurch gelöst, dass die Wand wellenförmig faltbar ist und in einer Dichtstellung derart wellenförmig ohne Knicke mit in Richtung auf den ersten Rand hin vorhandenen Faltlinien gefaltet ist, dass der erste Rand eine Wellenlinie definiert, welche vollständig auf einer Zylinderfläche liegt.

Üblicherweise wird bei chirurgischen Dichtungselementen die Dichtfunktion über eine ringförmige elastische Dichtlippe erzielt. Ein maximaler Durchmesser einer solchen Dichtlippe wird begrenzt durch den kleinsten Querschnitt im ungedehnten Zustand. Wird eine solche Dichtlippe über ihren maximal möglichen Durchmesser hinaus aufgedehnt, kann es zu Beschädigungen der Dichtungen sowie zu großen Spreizkräften und folglich zu hohen Reibkräften am Instrumentenschaft kommen. Ein erfindungsgemäß ausgebildetes Dichtelement zeichnet sich dadurch aus, dass der erste Rand nicht wie bei einer oben beschriebenen Dichtlippe ringförmig um einen Instrumentenschaft herum anliegt, sondern eine Wellenlinie auf einem Instrumentenschaft beschreibt. Wird ein Instrument mit einem größeren Durchmesser in das Dichtelement eingeführt, wird der erste Rand nicht wie bei der oben beschriebenen Dichtlippe elastisch gedehnt, sondern die gefaltete Wand entfaltet sich sukzessive. Dies hat zur Folge, dass die durch den ersten Rand definierte Wellenlinie in ihrer Gesamtbreite in axialer Richtung, die bestimmt wird durch einen Abstand von Wellentälern und Wellenbergen der Wellenlinie parallel zur Längsachse, kleiner wird. Eine sukzessive Entfaltung oder Glättung der Falten, die, wie beispielsweise bei einem Vorhang, knickfrei ausgebildet sind, führt somit zu einer teilweisen Verschiebung des ersten Randes in axialer Richtung. Insbesondere kann die Abdichtung eines Instrumentenschafts mit dem verbesserten Dichtelement ohne elastische Aufdehnung des ersten Randes und der Wand erzielt werden. Folglich sind auch die erforderlichen Spreizkräfte bei dem verbesserten Dichtelement im Vergleich zu aus dem Stand der Technik bekannten Dichtelementen verringert, ebenso Reibungskräfte beim Einführen des Instrumentenschafts in die Öffnung. Durch die auf Grund der Faltung definierte Wellenlinie, die beispielsweise sinusförmig verläuft oder aus Halbkreisabschnitten zusammengesetzt ist, des ersten Randes kann insgesamt ein weitaus größerer Bereich von Instrumentenschaftdurchmessern sicher abgedichtet werden, insbesondere ohne Dehnung des ersten Randes des Dichtelements. Zudem lässt sich das verbesserte Dichtelement auf Grund der vorgeschlagenen Faltung oder vorhangartigen Raffung der Wand in Richtung auf den ersten Rand hin bereits trichterförmig ausbilden, so dass das Einführen eines Instruments vereinfacht wird und Beschädigungen des Dichtelements durch das einzuführende Instrument verhindert werden. Des Weiteren wird ein Verschleiß gegenüber herkömmlichen, eine Dichtlippe aufweisenden Dichtelementen verringert, da beim erfindungsgemäß verbesserten Dichtelement keine Dehnung des ersten Randes erforderlich ist.

Vorzugsweise ist die Zylinderfläche konzentrisch zur Längsachse orientiert. So ist es auf einfache Weise möglich, zylindrische Instrumentenschäfte durch das Dichtelement einzuführen. Ferner kann so auch ein insgesamt rotations- oder punktsymmetrisches Dichtelement ausgebildet werden.

Günstig ist es, wenn das Dichtelement in einer Grundstellung eine Dichtungsstellung einnimmt, in der die Öffnung vollständig einen minimalen Durchmesser aufweist. Insbesondere kann die Öffnung geschlossen sein, also ein minimaler Durchmesser kann auch 0 mm betragen. Des Weiteren kann die Dichtung in der Grundstellung so ausgebildet sein, dass die Wand in Richtung auf den ersten Rand hin wellenförm ig gefaltet oder eingeschlagen ist, wie beispielsweise bei einem Vorhang. Dadurch wird das Einführen des Instruments erleichtert und eine Beschädigung des Dichtelements vermieden.

Vorteilhaft ist es, wenn das Dichtelement in der Grundstellung eine in Richtung auf den ersten Rand hin wellenförmig ohne Knicke gefaltete Wand aufweist und wenn der erste Rand eine Wellenlinie definiert, welche vollständig auf einer Zylinderfläche liegt. Ausgehend von dieser Grundstellung kann beim Einführen von Instrumentenschäften mit einem Schaftdurchmesser, der größer ist als der in der Grundstellung definierte minimale Innendurchmesser der Öffnung, eine Abdichtung des Instrumentenschafts allein dadurch erreicht werden, dass die Wand, ohne sie zu dehnen, entfaltet wird zum Erweitern der Öffnung.

Gemäß einer bevorzugten Ausführungsform der Erfindung kann ferner vorgesehen sein, dass die Wellenlinie oberhalb einer senkrecht zur Längsachse verlaufenden Öffnungsebene der Öffnung Wellenberge und unterhalb der Öffnung Wellentäler aufweist. Bei herkömmlichen Dichtelementen mit elastisch dehnbaren kreisförmigen Dichtlippen bestimmt die Dichtlippe die Öffnungsebene.

Bei dem erfindungsgemäß vorgeschlagenen Dichtelement liegt der erste Rand jedoch teilweise oberhalb und teilweise unterhalb der Öffnungsebene am Instrumentenschaft an beziehungsweise liegt auf einer virtuellen zylindrischen Fläche.

Vorteilhafterweise liegt ein Winkelabstand zwischen zwei Wellenbergen in einem Winkelbereich von 25° bis 50° bezogen auf die Längsachse. Dies bedeutet mit anderen Worten, dass die durch den ersten Rand gebildete Wellenlinie jeweils etwa sechs bis 15 Wellenberge und Wellentäler umfasst. So wird das Auffalten der Wand ausgehend vom ersten Rand zum Abdichten von Instrumentenschäften mit größeren Querschnitten deutlich erleichtert.

Günstig ist es, wenn die Wand in einer Vielzahl von Dichtstellungen mit jeweils unterschiedlichen Durchmessern der Öffnung in Richtung auf den ersten Rand hin derart wellenförmig ohne Knicke gefaltet ist, dass der erste Rand jeweils eine Wellenlinie definiert, welche vollständig auf einer Zylinderfläche liegt. Dadurch können auch Instrumentenschäfte abgedichtet werden, die beispielsweise einen sich konisch erweiternden oder verjüngenden Schaft aufweisen, denn jeder beliebige Schaftdurchmesser kann mit einem solchen erfindungsgemäßen Dichtelement sicher und zuverlässig abgedichtet werden.

Gemäß einer bevorzugten Ausführungsform der Erfindung kann vorgesehen sein, dass in einer maximalen Öffnungsstellung, in der die Wand faltenfrei und ein Durchmesser der Öffnung maximal ist, ein Durchmesser des ersten Randes maximal so groß ist wie ein Durchmesser des zweiten Randes.

Vorteilhafterweise kann die Wand in der maximalen Öffnungsstellung die Form einer dünnen zylindrischen Hülse aufweisen. Damit kann maximal ein Instrumentenschaft abgedichtet werden, dessen Außendurchmesser dem maximalen Durchmesser der Öffnung in der maximalen Öffnungsstellung entspricht.

Bei einer alternativen Ausführungsform kann es jedoch vorteilhaft sein, wenn die Wand in der maximalen Öffnungsstellung die Form eines flachen Kreisrings aufweist. Diese Ausgestaltung kann vorzugsweise bei Abdichtungssystemen zur Anwendung kommen, die nur eine minimale Bauchhöhe aufweisen dürfen.

Auch wenn es grundsätzlich nicht erforderlich ist, ist es trotzdem günstig, wenn der erste Rand in Form einer Dichtlippe ausgebildet ist. So kann insbesondere eine Abdichtung des ersten Rands an einem Instrumentenschaft weiter verbessert werden.

Günstigerweise trägt der erste Rand eine Dichtlippe. So kann beispielsweise die Dichtlippe aus einem anderen Material hergestellt sein als die Wand und damit der erste Rand noch besser an einem Instrumentenschaft anliegen, um die Öffnung relativ zum Instrumentenschaft abzudichten.

Besonders einfach wird der Aufbau des Dichtelements, wenn die Dichtlippe in Form eines Dichtrings oder in Form eines im Querschnitt kreisförmigen oder im Wesentlichen kreisförmigen Wulstes ausgebildet ist. Die Dichtlippe ist dabei vorzugsweise so ausgebildet, dass sie ebenfalls die Form einer Wellenlinie entsprechend des ersten Randes annimmt.

Um eine besonders gute Abdichtung zu erzielen, ist die Dichtlippe günstigerweise aus einem elastischen dehnfähigen Material hergestellt. Beispielsweise kann die Dichtlippe in Form eines am ersten Rand angeformten oder mit diesem verbundenen O-Rings ausgebildet sein.

Vorteilhafterweise sind die Wand und die Dichtlippe aus unterschiedlichen Materialien hergestellt. Dies ermöglicht es, die Wand vorzugsweise inelastisch auszubilden, die Dichtlippe dagegen elastisch.

Gemäß einer bevorzugten Ausführungsform der Erfindung kann vorgesehen sein, dass die Dichtlippe aus einem Material hergestellt ist, das einen Elastizitätsmodul *E* aufweist, der um einen Faktor 10² bis 10⁵ größer ist als ein Elastizitätsmodul des Materials, aus dem die Wand hergestellt ist. Die Dichtlippe ist somit aus einem dehnfähigeren Material hergestellt als die Wand, so dass sich die Dichtlippe elastisch um einen Instrumentenschaft herum anlegen kann, die Wand jedoch praktisch inelastisch ist und nicht gedehnt werden kann.

Vorzugsweise ist die Wand aus einem inelastischen oder im Wesentlichen inelastischen Material hergestellt. Dies hat insbesondere den Vorteil, dass Materialien verwendet werden können, die es gestatten, dass die Wand dauerhaft ihre durch die Herstellung vorgegebene Form beibehält, insbesondere die Form, die sie in der Grundstellung aufweist.

Vorzugsweise ist das Dichtelement einstückig ausgebildet. Dies hat den Vorteil, dass es in einem einzigen Arbeitsschritt hergestellt werden kann.

Günstig ist es, wenn das Dichtelement und/oder die Dichtlippe aus mindestens einem Kunststoff hergestellt sind. Das Dichtelement kann so besonders kostengünstig hergestellt werden.

Vorteilhafterweise ist der Kunststoff Gummi, Silikon oder ein Silikon und/oder Gummi enthaltender Kunststoff. Einen derartigen Kunststoff zu verwenden hat insbesondere den Vorteil, dass die Wand des Dichtelements ausreichend flexibel ist, um in Falten gelegt oder gerafft zu werden und um sich auch wieder entfalten zu können.

Besonders kostengünstig wird das Dichtelement ferner dann, wenn es durch Spritzgießen hergestellt ist.

Gemäß einer bevorzugten Ausführungsform der Erfindung kann vorgesehen sein, dass der zweite Rand in jeder Dichtstellung derart geformt und ausgebildet ist, dass er vollständig in einer quer zur Längsachse verlaufenden Ebene liegt. Beispielsweise kann der zweite Rand kreisförmig oder oval ausgebildet sein. Dies bedeutet, dass die Wand des Dichtelements an einem Ende kreisförmig und ungefaltet sein kann, wohingegen sie an ihrem anderen, den ersten Rand bildenden Ende durch die vorgesehene Faltung die Form einer auf einer Zylinderfläche liegenden Wellenlinie aufweist.

Um das Festlegen des Dichtelements an einem Trokar zu erleichtern, ist es günstig, wenn der zweite Rand derart geformt und ausgebildet ist, dass er an einem Rahmen oder an einer Halterung eines Trokars festlegbar ist.

Vorzugsweise ist eine unflexible Halterung vorgesehen und der zweite Rand in der unflexiblen Halterung gehalten. Dies hat den Vorteil, dass beim Verbinden des Dichtelements mit einem Trokar das Dichtelement nicht beschädigt werden kann. Zudem kann die Halterung in gewünschter Weise an eine dafür vorgesehene Aufnahme des Trokars angepasst sein. Die Halterung bildet somit quasi ein Interface zwischen dem Dichtelement und dem Trokar.

Besonders einfach wird der Aufbau des Dichtelements, wenn die Halterung einen Haltering umfasst. Dieser kann beispielsweise mit dem zweiten Rand oder im Bereich des zweiten Randes angeschweißt oder angeklebt sein. Denkbar wäre es auch, die Halterung mit dem zweiten Rand klemmend zu verbinden oder im Bereich des zweiten Randes klemmend festzulegen.

Vorzugsweise ist die Halterung aus einem anderen Material hergestellt als das Dichtelement. Dies ermöglicht es, für die Halterung insbesondere ein steiferes Material zur Herstellung zu verwenden als für das Dichtelement.

Günstig ist es, wenn die Halterung aus einem Kunststoff oder einem Metall hergestellt ist. Eine aus einem Kunststoff oder aus einem Metall hergestellte Halterung weist die erforderliche Stabilität auf, um das Dichtelement sicher zu halten, insbesondere an einem Trokar.

Je nachdem, aus welchem Material das Dichtelement hergestellt ist, kann das Einführen eines Instruments durch die Öffnung des Dichtelements erschwert sein. Daher ist es vorteilhaft, wenn das Dichtelement mindestens teilweise mit einer reibungsmindernden Beschichtung versehen ist. Selbstverständlich kann das Dichtelement auch vollständig mit einer reibungsmindernden Beschichtung versehen sein, die das Einführen eines Instruments durch die Öffnung des Dichtelements erleichtert.

Um die Flexibilität der Wand nicht unnötig zu beeinträchtigen, ist es günstig, wenn die reibungsmindernde Beschichtung mindestens teilweise auf einer Innenseite der Wand aufgebracht ist. In der Regel kommt es nur zwischen einer Innenseite der Wand und einem Instrument, das durch die Öffnung des Dichtelements eingeführt ist, zu Reibungsverlusten. Wenn nur die Innenseite der Wand mit einer reibungsmindernden Beschichtung versehen wird, hat dies den Vorteil, dass nur dort, wo die Beschichtung üblicherweise benötigt wird, eine solche Beschichtung vorgesehen werden muss.

Gemäß einer bevorzugten Ausführungsform der Erfindung kann vorgesehen sein, dass die reibungsmindernde Beschichtung aus Polytetrafluorethylen (PTFE) hergestellt ist oder Polytetrafluorethylen (PTFE) enthält. Insbesondere eine mit einer PTFE-Beschichtung versehene Innenseite der Wand des Dichtelements verringert Reibungsverluste beim Einführen von Instrumenten, deren Schaftdurchmesser größer sind als ein Innendurchmesser der Öffnung des Dichtelements in der Grundstellung.

Um das Dichtelement auf einfache Weise, beispielsweise klemmend in einer Halterung oder an einem Trokar festzulegen, ist es vorteilhaft, wenn der zweite Rand wulstförm ig ausgebildet ist. Vorzugsweise steht der zweite Rand wulstförmig radial von der Längsachse weg weisend nach außen vor.

Vorteilhaft ist es, wenn der zweite Rand in Richtung oder im Wesentlichen in Richtung auf den ersten Rand hin umgeschlagen ist und wenn eine Dichtfläche im Bereich des zweiten Randes ausgebildet ist, die vom ersten Rand weg weist. Der umgeschlagene zweite Rand ermöglicht es insbesondere, einen Haltering in die ausgebildete ringförmige Ausnehmung einzulegen und das Dichtelement zwischen dem Haltering und einer Innenwand eines Trokars klemmend festzulegen. Ferner wird so auf einfache Weise eine Dichtfläche definiert, die vorzugsweise eben ist.

Günstigerweise verläuft die Dichtfläche in einer Ebene quer zu einer Längsachse des Dichtelements. Dies gestattet es, das Dichtelement beispielsweise gegen eine ringförmige Dichtfläche eines Flansches zu halten, um eine Einführöffnung eines Trokars abzudichten.

Auf einfache Weise lässt sich das Dichtelement mit einem weiteren Bauteil, beispielsweise einem Trokar verbinden, wenn im Bereich des zweiten Randes ein radial oder im Wesentlichen radial von der Längsachse weg weisender Flansch ausgebildet, angeordnet oder angeformt ist. Beispielsweise kann der Flansch aus einem anderen Material hergestellt sein als die Wand des Dichtelements. Insbesondere dann ist es günstig, wenn der Flansch mit der Wand verklebt oder verschweißt ist.

Die eingangs gestellte Aufgabe wird ferner bei einer chirurgischen Dichtung der eingangs beschriebenen Art erfindungsgemäß dadurch gelöst, dass die Wand wellenförmig faltbar ist und in einer Dichtstellung derart wellenförmig ohne Knicke mit in Richtung auf den ersten Rand hin verlaufenden Faltlinien gefaltet ist, dass der erste Rand eine Wellenlinie definiert, welche vollständig auf einer Zylinderfläche liegt, dass ein zweites, identisch oder im Wesentlichen identisch mit dem ersten Dichtelement ausgebildetes Dichtelement mit einer zweiten Wand, die auch einen ersten und einen zweiten Rand aufweist, vorgesehen ist und dass der erste Rand des ersten Dichtelements mit dem ersten Rand des zweiten Dichtelements verbunden ist, so dass in der ersten Dichtstellung ein Wellenberg einer durch den ersten Rand des ersten Dichtelements definierten Wellenlinie in ein Wellental einer durch den ersten Rand des zweiten Dichtelements definierten Wellenlinie eingreift.

Zwei derart geformte Dichtelemente lassen sich auf einfache Weise vollständig entlang ihrer ersten Ränder verbinden, indem die beiden Dichtelemente relativ zueinander um ihre gemeinsame Längsachse um einen Winkelabstand verdreht werden, die einem Abstand zwischen zwei Wellenbergen der Wellenlinie einer der beiden ersten Ränder entspricht. Beispielsweise können die beiden Dichtelemente miteinander verklebt oder verschweißt werden. Denkbar wäre es auch, die chirurgische Dichtung einstückig herzustellen. Die erfindungsgemäß weitergebildete chirurgische Dichtung ermöglicht es, ein chirurgisches Instrument von beiden Seiten einzuführen, und stellt eine Abdichtung zu einem chirurgischen Instrument in gewünschter Weise sicher. Des Weiteren kann so vermieden werden, dass sich der erste Rand eines einzelnen Dichtelements beim Einführen oder Herausziehen eines Instruments, je nachdem, ob es zuerst durch die vom ersten Rand gebildete Öffnung oder durch eine vom zweiten Rand gebildete Öffnung eingeführt wird, umstülpt. Die Position des ersten Randes wird somit auf einfache Weise dauerhaft festgelegt. Ferner wird die Stabilität der Dichtung im Vergleich zu einem einzelnen Dichtelement, wie es oben beschrieben wurde, erhöht, allerdings ohne Reibungskräfte beim Einführen eines Instrumentenschafts durch die Öffnung zu erhöhen, da lediglich die Wand entfaltet, jedoch nicht gedehnt werden muss. Dadurch werden unabhängig davon, wie groß ein Durchmesser eines Instrumentenschafts ist, im Wesentlichen stets die gleichen Kräfte benötigt, um beim Einführen des Instrumentenschafts in die Dichtung die Öffnung des Dichtelements zu vergrößern.

Vorzugsweise ist die Dichtung doppeltrichterförmig ausgebildet, das heißt die den beiden die Dichtung ausbildenden Dichtelementen gemeinsame Öffnung ist kleiner als eine durch die zweiten Ränder definierte Öffnung, so dass sich eine Wand der beiden Dichtelemente in Richtung auf die gemeinsame Öffnung hin verjüngt.

Gemäß einer weiteren bevorzugten Ausführungsform der Erfindung kann vorgesehen sein, dass das erste und/oder das zweite Dichtelement Dichtelemente der oben beschriebenen Art sind.

Ferner wird die eingangs gestellte Aufgabe bei einem chirurgischen Abdichtungssystem der eingangs beschriebenen Art erfindungsgemäß dadurch gelöst, dass das Dichtelement eines der oben beschriebenen Dichtelemente ist oder dass das Dichtelement eine der oben beschriebenen chirurgischen Dichtungen ist.

Wie im Zusammenhang mit den oben beschriebenen Dichtelementen sowie der oben beschriebenen Dichtungen erläutert, kann mit einem erfindungsgemäß vorgeschlagenen chirurgischen Abdichtungssystem eine Vielzahl langgestreckter chirurgischer Instrumente mit beliebigen Schaftdurchmessern in das Abdichtungssystem eingeführt und von diesem abgedichtet werden.

Vorteilhafterweise ist das Dichtelement mit dem Trokar lösbar verbindbar. Dies ermöglicht es, nur das Dichtelement auszutauschen, wenn dieses beschädigt wurde oder gealtert ist.

Die nachfolgende Beschreibung bevorzugter Ausführungsformen der Erfindung dient im Zusammenhang mit der Zeichnung der näheren Erläuterung. Es zeigen:
- Figur 1:: eine teilweise geschnittene Seitenansicht eines erfindungsgemäßen chirurgischen Abdichtungssystems umfassend ein erstes Ausfüh- rungsbeispiel eines erfindungsgemäßen Dichtelements;
- Figur 2:: eine perspektivische Ansicht des ersten Ausführungsbeispiels eines erfindungsgemäßen Dichtelements aus Figur 1;
- Figur 3:: eine Seitenansicht des Dichtelements aus Figur 2;
- Figur 4:: eine Schnittansicht des Dichtungselements aus Figur 3 längs Linie 4-4 mit eingesetztem Instrumentenschaft;
- Figur 5a:: eine Draufsicht auf ein erfindungsgemäßes Dichtelement in der Grundstellung;
- Figur 5b:: eine Draufsicht auf das Dichtelement aus Figur 5a in einer Dicht- stellung mit gegenüber der Grundstellung vergrößerter Öffnung;
- Figur 6:: eine Seitenansicht eines zweiten Ausführungsbeispiels eines erfin- dungsgemäßen Dichtelements mit eingeschobenem Instrumenten- schaft;
- Figur 7:: eine perspektivische Ansicht eines dritten Ausführungsbeispiels eines erfindungsgemäßen Dichtelementes; und
- Figur 8:: eine perspektivische Ansicht eines vierten Ausführungsbeispiels ei- nes erfindungsgemäßen Dichtelements.

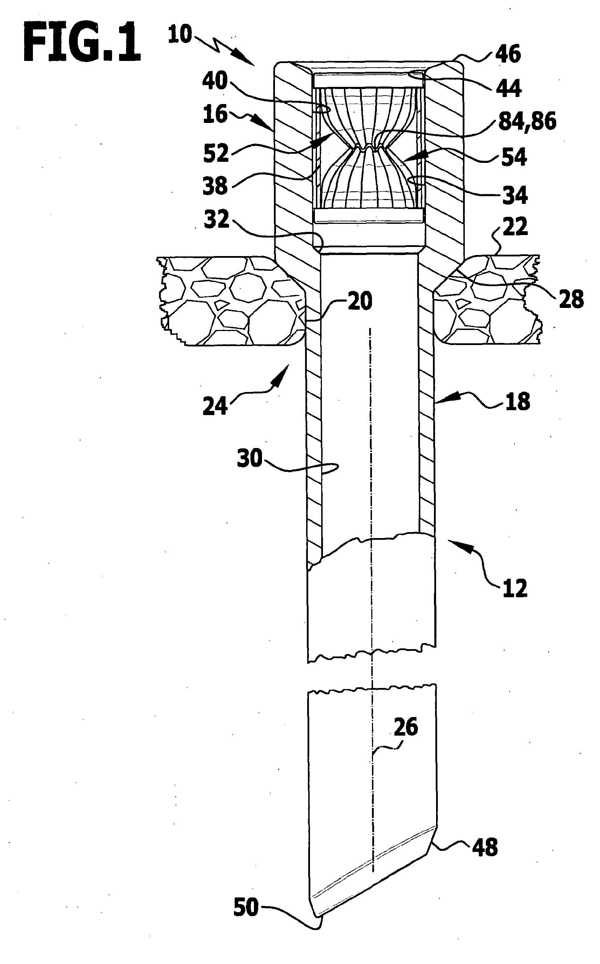

In Figur 1 ist ein insgesamt mit dem Bezugszeichen 10 versehenes chirurgisches Abdichtungssystem dargestellt, welches einen langgestreckten, hülsenförmigen Trokar 12 mit einer chirurgischen Dichtung umfasst.

Der Trokar 12 umfasst einen einen proximalen Endabschnitt des Trokars 12 bildenden Dichtungsabschnitt 16, an den sich distalseitig ein langgestreckter Einführabschnitt 18 anschließt, der durch eine Inzision 20 in menschlichem oder tierischem Gewebe, beispielsweise einer Haut 22, in einen Hohlraum 24 eines menschlichen oder tierischen Körpers, beispielsweise einen Bauchraum, eingeführt werden kann. Ein Außendurchmesser des Einführabschnitts 18 ist etwas kleiner als ein Außendurchmesser des Dichtungsabschnitts 16, so dass im Übergangsbereich zwischen beiden Abschnitten eine ringförmige, relativ zu einer Längsachse 26 des Trokars 12 etwa um 45° geneigte Ringfläche 28 ausgebildet ist, die im Wesentlichen in distaler Richtung weist. Die Ringfläche 28 bildet insbesondere eine Anschlagfläche, um zu verhindern, dass der Dichtungsabschnitt 16 durch die Inzision 20 in den Hohlraum 24 hineingleiten kann. In ähnlicher Weise erweitert sich im Übergangsbereich vom Einführabschnitt 18 zum Dichtungsabschnitt 16 ein Innendurchmesser einer sich durch den gesamten Trokar 12 konzentrisch zur Längsachse 26 erstreckenden Bohrung 30 einstufig, so dass eine zweite Ringfläche 32 ausgebildet ist, die ebenfalls um etwa 45° bezogen auf die Längsachse 26 geneigt ist und im Wesentlichen in proximaler Richtung weist.

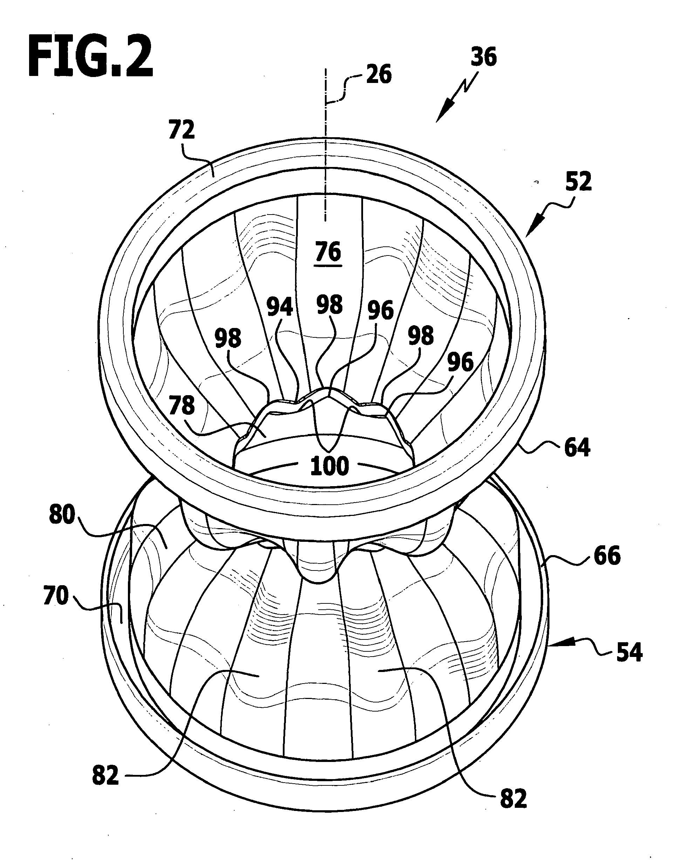

Die sich im Bereich des Dichtungsabschnitts 16 einstufig erweiternde Bohrung 30 bildet eine Dichtungsaufnahme 34 für eine insgesamt mit dem Bezugszeichen 36 versehene chirurgische Dichtung, die nachfolgend im Zusammenhang mit den Figuren 2 bis 5 näher erläutert wird. Des Weiteren ist in der Dichtungsaufnahme 34 eine Halterung 38 in Form einer von einer Innenwand 40 des Dichtungsabschnitts 16 etwas beabstandet und konzentrisch zur Längsachse 26 angeordneten Haltehülse, so dass zum indest im Bereich der beiden Enden ein Spalt 42 zwischen der Halterung 38 und der Innenwand 40 ausgebildet ist. Vorzugsweise ist die Halterung 38 aus einem harten und sterilisierbaren Kunststoff, wie zum Beispiel Polyetheretherketon (PEEK), oder aus einem Metall hergestellt.

Die Dichtung 36 kann auf einfache Weise durch eine Einführöffnung 44 ausgehend von einem proximalen Ende 46 des Trokars 12 in die Dichtungsaufnahme 34 eingeschoben werden. Durch die Einführöffnung 44 kann nach Festlegen der Dichtung 36 in der Dichtungsaufnahme 34 ein nicht dargestelltes endoskopisches Instrument mit einem langgestreckten Instrumentenschaft eingeführt werden. Die nachfolgend im Einzelnen näher beschriebene Dichtung 36 stellt eine fluiddichte Abdichtung zwischen dem Trokar 12 und einem Schaft 102 des eingeführten Instruments sicher.

Um das Einführen des Trokars 12 in einen menschlichen oder tierischen Körper zu erleichtern und um eine Verletzung von Gewebe zu vermeiden, ist ein distales Ende des Einführabschnitts 18 auf einem kurzen Abschnitt mit einer sich in distaler Richtung hin konisch verjüngenden Außenfläche 48 versehen, die zudem relativ zur Längsachse etwas geneigt ist, so dass eine ringförmige Stirnfläche 50 des Einführabschnitts 18 unter einem Winkel von etwa 30° schräg in distaler Richtung weist.

Der Aufbau der Dichtung 36 wird nachfolgend im Zusammenhang mit den Figuren 2 bis 5 näher erläutert.

Die Dichtung 36 wird gebildet durch zwei identische Dichtungselemente 52 und 54, die miteinander verbunden oder einstückig zur Ausbildung der Dichtung 36 ausgebildet sind. Jedes Dichtelement 52 beziehungsweise 54 weist eine in sich geschlossene ringförmige flexible Wand 56 beziehungsweise 58 auf, welche einen ersten Rand 60 beziehungsweise 62 sowie einen zweiten Rand 64 beziehungsweise 66 aufweisen. Die ersten Ränder 60 und 62 bilden jeweils ein erstes Ende der Dichtungselemente 52 und 54, die zweiten Ränder 64 und 66 ein zweites Ende der Dichtungselemente 52 und 54. Die zweiten Ränder 64 und 66 sind nach außen umgeschlagen, so dass sie im Wesentlichen in Richtung auf die ersten Ränder 60 beziehungsweise 62 weisen und im Wesentlichen in Richtung auf die ersten Ränder 60 beziehungsweise 62 hin geöffneten Ringnute 68 und 70 ausbilden, in die die hülsenförmige Halterung 38 am Trokar 12 eingeschoben werden kann. Die zweiten Ränder 64 und 66 sind vorzugsweise kreisförm ig ausgebildet und liegen in einer Ebene quer zur Längsachse 26. Durch das Umschlagen der zweiten Ränder 64 und 66 bildet ein Teil der Innenseiten 76 und 78 der Wände 56 und 58 kreisringförmige Dichtflächen 72 und 74, die jeweils vom ersten Rand 60 beziehungsweise 62 weg weisen und deren Flächennormale parallel zur Längsachse 26 verläuft.

Die Wände 56 und 58 sind im Bereich der Dichtflächen 72 und 74 im Querschnitt kreisförmig. In Richtung auf die ersten Ränder 60 und 62 hin sind sie wellenförmig ohne Knicke mit in Richtung auf die ersten Ränder 60 und 62 verlaufenden Faltlinien gefaltet. Anders ausgedrückt sind die Wände 56 und 58 in Richtung auf die ersten Ränder 60 und 62 hin vorhangartig gerafft, so dass die ersten Ränder 60 und 62 in einer Seitenansicht eine Wellenlinie 84 beziehungsweise 86 definieren. In der Seitenansicht sind somit die ersten Ränder 60 und 62 wellenförmig ausgebildet, und zwar in etwa sinusförmig. Dagegen begrenzen sie in einer Draufsicht in Richtung der Längsachse 26 jeweils eine kreisringförmige Öffnung 88 beziehungsweise 90. Die Wellenlinien 84 und 86 verlaufen derart, dass in einer senkrecht zur Längsachse 26 verlaufenden Öffnungsebene 92 Wellenberge 94 und 96 vom jeweils zweiten Rand 64 beziehungsweise 66 aus gesehen auf einer anderen Seite der Öffnungsebene 92 verlaufen, Wellentäler 98 und 100 der Wellenlinien 84 und 86 dagegen auf der gleichen Seite wie die zweiten Ränder 64 und 66 bezogen auf die Öffnungsebene 92.

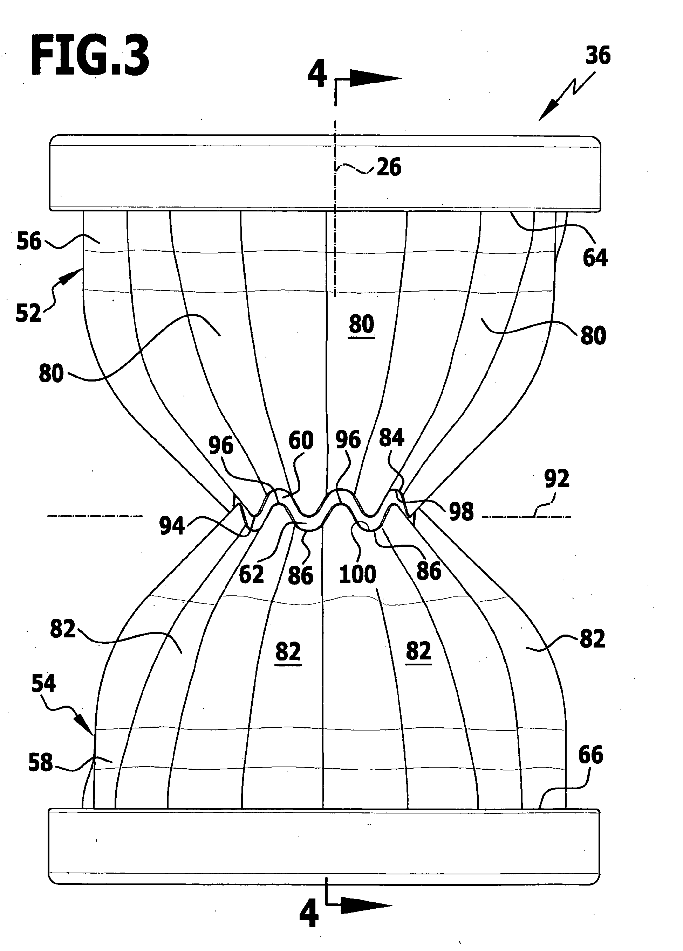

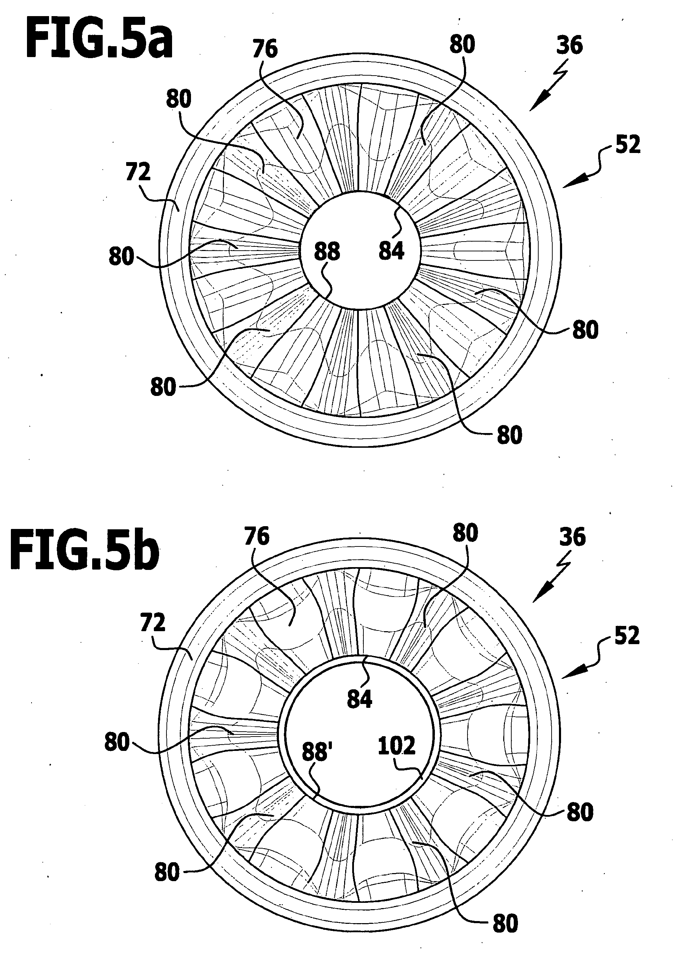

Jedes der beiden Dichtelemente 52 und 54 ist somit im Wesentlichen trichterförmig geformt und verjüngt sich in einem Innendurchmesser in Richtung auf den ersten Rand 60 und 62 hin. Die ersten Ränder 60 und 62, obwohl sie in axialer Richtung jeweils eine Wellenlinie 84 beziehungsweise 86 definieren, wie dies in der Seitenansicht in Figur 3 dargestellt ist, liegen jedoch vollständig auf einer die Längsachse 26 konzentrisch umgebenden Zylinderfläche. Damit ergibt sich insgesamt eine in der in Figur 5a dargestellten Draufsicht kreisförmige Öffnung 88.

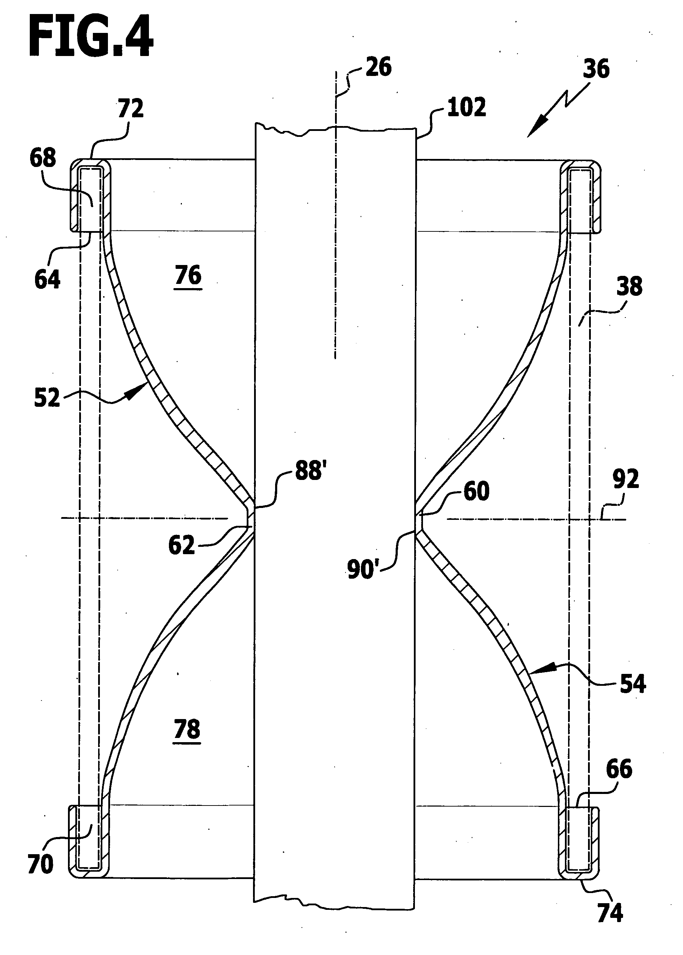

Wie in den Figuren 1 bis 4 dargestellt, wird die Dichtung 36 durch die beiden Dichtelemente 42 und 44 derart gebildet, dass die ersten Ränder 60 und 62 miteinander verbunden sind, was dadurch erreicht wird, dass die beiden Dichtelemente 52 und 54 relativ zueinander um die Längsachse um einen halben Abstand zwischen zwei Wellenbergen 94 beziehungsweise 96 oder zwei Wellentälern 98 beziehungsweise 100 verdreht sind, so dass die Wellenberge 94 des Dichtelements 52 in die Wellentäler 100 des Dichtelements 54 eintauchen und die Wellenberge 96 des zweiten Dichtelements 54 in die Wellentäler 98 des Dichtelements 52. Damit liegen die ersten Ränder 60 und 62 vollständig aneinander an und definieren eine gemeinsame Wellenlinie 84 beziehungsweise 86. Die Dichtung 36 ist damit insgesamt doppeltrichterförmig ausgebildet und verjüngt sich in Richtung auf die Öffnungsebene 92 hin. Die Ringnuten 68 und 70 weisen aufeinander zu, so dass die Dichtung auf der zylinderhülsenförmigen Halterung 38 gehalten ist, die beidseitig in die Ringnuten 68 und 70 eintaucht, wie in Figur 4 dargestellt. Die miteinander verbundenen ersten Ränder 60 und 62 bilden somit eine Art Dichtlippe der Dichtung 36 aus, die in einer Seitenansicht in Form einer Wellenlinie 84 beziehungsweise 86 verläuft, jedoch vollständig auf einer Zylinderfläche liegt und somit ein im Querschnitt kreisförmiges Instrument oder einen Schaft 102 vollständig dicht umschließen kann.

Grundsätzlich wäre es zwar denkbar, die Wände 56 und 58 aus einem elastisch dehnbaren Material herzustellen. Dies ist jedoch für die vorliegende Erfindung nicht erforderlich. Es genügt, wenn die Wand aus einem flexiblen Material hergestellt ist. Dann ist es nämlich möglich, ein chirurgisches Instrument mit einem langgestreckten zylindrischen Schaft 102 in die Dichtung 36 einzuführen und abzudichten. Ist ein Außendurchmesser des Schafts 102 größer als der Innendurchmesser der Öffnungen 88 und 90 in der Grundstellung, dann führt der Schaft 102 zu einer Auffaltung der Wände 56 und 58, jedoch liegen die ersten Ränder 60 und 62 immer noch vollständig am Schaft 102 an, da sie in jeder Dichtstellung vollständig auf einer Zylinderfläche liegen. Eine veränderte Faltung durch den eingeführten Schaft 102 ist in Figur 5b dargestellt. Die im Durchmesser erweiterte Öffnung 88' ist immer noch kreisförmig, wobei die ersten Ränder 60 und 62 weiterhin eine Wellenlinie 84 beziehungsweise 86 definieren. Allerdings sind die Wellenberge 94 und 96 sowie die Wellentäler 98 und 100 nicht mehr so hoch wie bei der die Grundstellung einnehmenden Dichtung 36. Die Abdichtung der Instrumente wird somit nur durch ein Entfalten der Wände 56 und 58 erreicht, nicht jedoch durch eine Dehnung der ersten Ränder 60 und 62 beziehungsweise der Wände 56 und 58. Dies hat den Vorteil, dass beim Einführen eines Instruments mit einem Schaft 102, der im Durchmesser größer ist als ein Innendurchmesser der Öffnung 88 der Dichtung 36 in der Grundstellung, eine geringere Kraft aufgewendet werden muss, um das Instrument in den Trokar 12 einzuführen, als dies bei herkömmlichen Trokaren mit Dichtungen der Fall ist, die auf dem Prinzip der elastischen Dehnung einer Dichtlippe oder eines Dichtungsrandes beruhen.

Die erfindungsgemäß vorgeschlagenen Dichtelemente 52 und 54 und somit auch die Dichtung 36 ermöglichen es im Extremfall, ein Instrument mit einem Außendurchmesser einzuführen, der einem Innendurchmesser der Dichtelemente 52 und 54 im Bereich des umgeschlagenen zweiten Randes 64 beziehungsweise 66 entspricht, denn die ersten Ränder 60 und 62 sind vorzugsweise so gefaltet, dass sie in ungefaltetem oder ungerafftem Zustand keine Wellenlinie 84 beziehungsweise 86 mehr definieren, sondern eine die Längsachse 26 konzentrisch umgebende Kreislinie. Dies bedeutet jedoch aber auch, dass in Folge einer Auffaltung der Wände 56 und 58 der Dichtelemente 52 und 54 sich ein Abstand zwischen der Öffnungsebene 92 und den Dichtflächen 72 und 74 ändert. Ein minimaler Abstand wird in der Grundstellung vorgegeben, in der die Öffnung 88 einen minimalen Innendurchmesser aufweist. In einer maximal aufgefalteten Stellung der Dichtung 36 sind die Abstände der Dichtflächen 72 und 74 von der Öffnungsebene 92 maximal. Dies bedeutet jedoch aber auch, dass die Dichtung 36 vorzugsweise derart am Trokar 12 gehaltert sein sollte, dass die beiden Dichtflächen 72 und 74 relativ zueinander in axialer Richtung beweglich gehalten sind.

Durch die doppeltrichterförmige Ausbildung der Dichtung 36 können Schäfte 102 von beiden Seiten in die Dichtung 36 eingeführt werden. Ein Zurückziehen des Schafts 102 aus der Dichtung 36 heraus führt nicht zu einem unerwünschten Umstülpen der ersten Ränder 60 und 62.

Die Dichtung 36 ist vorzugsweise einstückig ausgebildet und aus einem Kunststoff durch Spritzgießen hergestellt. Als Kunststoffe eignen sich insbesondere Gummi, Silikone oder Silikone und/oder Gummi enthaltende Kunststoffe, die optional mit einer reibungsmindernden Beschichtung aus Polytetrafluorethylen (PTFE) versehen sein können. Insbesondere kann die reibungsmindernde Beschichtung auf einer Innenseite der Wand 56 aufgebracht sein.

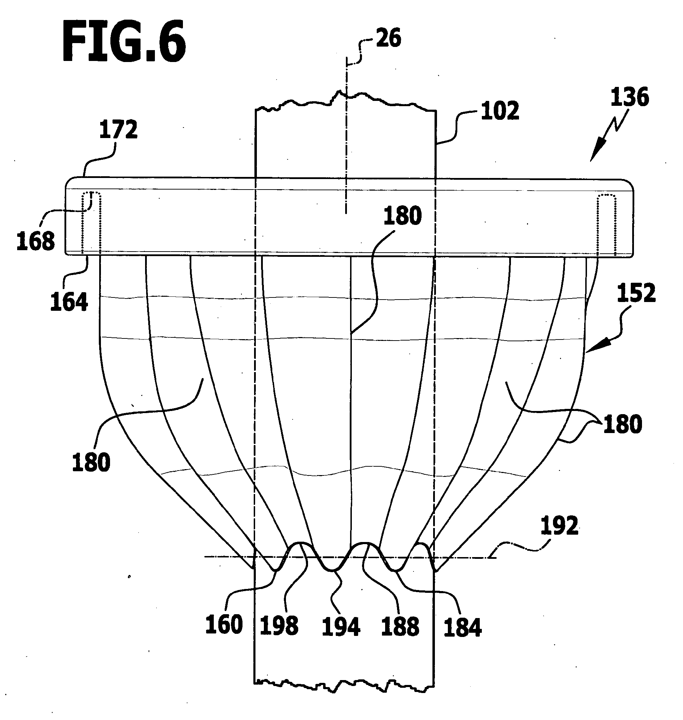

Ein zweites Ausführungsbeispiel einer insgesamt mit dem Bezugszeichen 136 versehenen Dichtung ist in Figur 6 mit einem eingeführten, abgedichteten Instrumentenschaft 102 dargestellt. Die Dichtung 136 wird nur aus einem einzigen Dichtelement 152 gebildet, das identisch mit dem Dichtelement 52 ausgebildet ist, so dass identische Teile des Dichtelements 152 mit Bezugszeichen versehen sind, die die selben beiden Endziffern aufweisen wie die entsprechenden Bezugszeichen des Dichtelements 52. Durch den knickfrei wellenförmig gefalteten oder gerafften ersten Rand 160 ergibt sich in der in Figur 6 dargestellten Seitenansicht eine am im Querschnitt kreisförmigen Schaft 102, also auf einer Zylinderfläche, anliegende Wellenlinie 184, die eine kreisförmige Öffnung 188 in einer Draufsicht definiert, wie sie in Figur 5b zu sehen ist. Insgesamt liegt die Wand 156 des Dichtelements 152 ebenso in Falten wie die Wand 56 des Dichtelements 52. Der zweite Rand 164 ist ebenfalls in Richtung auf den ersten Rand 160 hin umgeschlagen, so dass eine Ringnut 168 ausgebildet wird, in die beispielsweise ein Haltering oder eine Haltehülse eintauchen kann, um das Dichtelement 152 an einem Trokar festzulegen.

Die Wellenlinie 184 bildet, wie in Figur 6 in der Seitenansicht zu sehen, bezogen auf eine mittlere Öffnungsebene 192 Wellenberge 194 und Wellentäler 198 aus. Auch beim Dichtelement 152 ist es nicht erforderlich, die Wand aus einem elastisch dehnbaren Material herzustellen, es genügt, wenn die Wand 156 aus einem flexiblen Material hergestellt ist, welches es gestattet, die Öffnung 188 durch Entfalten und damit durch Verringerung einer Höhe beziehungsweise Tiefe der Wellenberge 194 und der Wellentäler 198 zu verändern.

Das Dichtelement 152 kann aus denselben Materialien hergestellt sein, wie die Dichtung 36.

Des Weiteren ist es möglich, im Bereich der ersten Ränder 60 und 62 der Dichtung 36 beziehungsweise des ersten Rands 160 der Dichtung 136 eine Dichtlippe auszubilden, die vorzugsweise in radialer Richtung in Richtung auf die Längsachse 26 hin weist. Die Dichtlippe kann einstückig an die jeweilige Dichtung 36 beziehungsweise 136 angeformt sein, kann jedoch auch aus einem anderen Material bestehen, insbesondere auch aus einem elastisch dehnbaren Material und mit dem jeweils ersten Rand 60 beziehungsweise 62 oder 160 verklebt oder verschweißt sein.

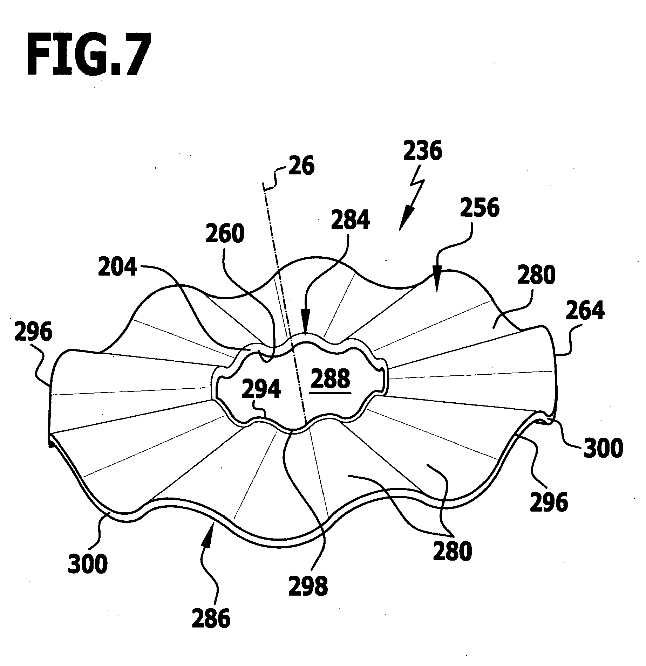

Ein drittes Ausführungsbeispiel einer erfindungsgemäßen Dichtung ist in Figur 7 dargestellt und insgesamt mit dem Bezugszeichen 236 versehen. Sie umfasst ebenfalls einen ersten Rand 260 sowie einen zweiten Rand 264. Der erste Rand 260 definiert eine in der Draufsicht kreisförmige Öffnung, ist jedoch in einer Seitenansicht ähnlich wie die ersten Ränder 60, 62 und 160 der Dichtungen 36 und 136 in Form einer Wellenlinie geformt. Die Wellenlinie liegt vollständig auf einer konzentrisch um die Längsachse 26 verlaufenden Zylinderfläche. Der zweite Rand 264 ist ebenfalls in Form einer Wellenlinie geformt und liegt, anders als die zweiten Ränder 64 und 66 der Dichtung 36, ebenfalls auf einer die Längsachse 26 konzentrisch umgebenden Zylinderfläche, deren Durchmesser jedoch etwa dreimal dem Durchmesser der Zylinderfläche entspricht, auf der der erste Rand 260 liegt. Die Wand 256 liegt folglich im Wesentlichen in einer Ebene, der sogenannten Öffnungsebene, oberhalb derer Wellenberge 294 des ersten Rands 260 und Wellenberge 296 des zweiten Rands 264 liegen. Unterhalb der Öffnungsebene liegen die Wellentäler 298 des ersten Rands 260 sowie die Wellentäler 300 des zweiten Rands 264. Die aus einem vorzugsweise inelastischen, jedoch flexiblen Material hergestellte Wand 256 liegt somit insgesamt in knickfreien Falten, die Faltenlinien 280 definieren, welche sich von der Längsachse 26 weg in radialer Richtung erstrecken. In einer völlig entfalteten Dichtstellung nimmt die Wand 156 die Form eines flachen Kreisrings an.

Bei der in Figur 7 dargestellten Dichtung 236 ist an dem ersten Rand 260 zusätzlich eine wulstförmige Dichtlippe 204 angeformt. Die Ausstattung einer Dichtung 236 mit einer Dichtlippe 204 ist jedoch optional und auf Grund des bereits oben näher beschriebenen Prinzips der Abdichtung eines zylindrischen Schafts nicht erforderlich. Die Dichtlippe 204 ist vorzugsweise aus einem elastischen Material hergestellt und steht in radialer Richtung auf die Längsachse 26 hin weisend vom ersten Rand 260 ab. Die Dichtlippe 204 kann an den ersten Rand 260 angespritzt, angeschweißt oder angeklebt sein.

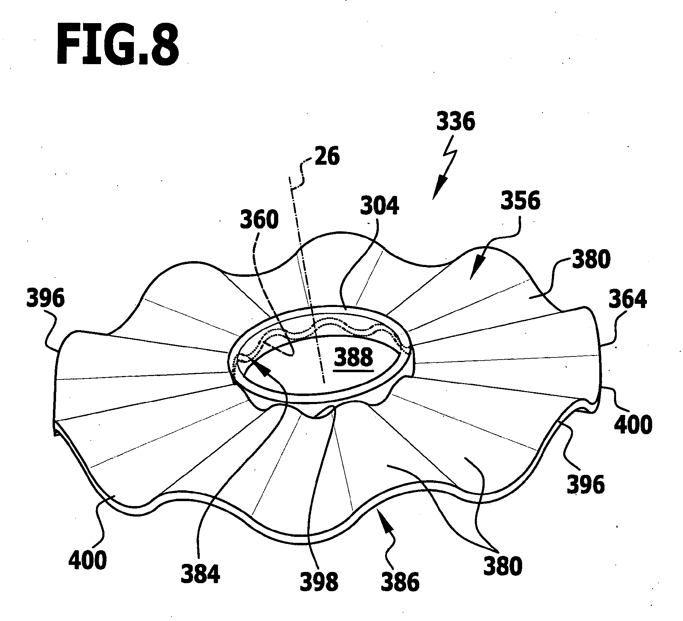

Ein viertes Ausführungsbeispiel einer erfindungsgemäßen Dichtung ist in Figur 8 insgesamt mit dem Bezugszeichen 336 versehen. Sie ist im Wesentlichen identisch mit der Dichtung 236 aufgebaut, so dass Teile der Dichtung 336, die Teilen der Dichtung 236 entsprechen, mit Bezugszeichen versehen sind, die die selben zwei Endziffern aufweisen.

Die Dichtung 336 ist som it ebenso wie die Dichtung 236 in einer Grundstellung, in der ein erster Rand 360 eine minimale Öffnung 388 mit einem minimalen Innendurchmesser definiert, so dass eine Wand 356 der Dichtung 336 im Wesentlichen eine Ebene, die sogenannte Öffnungsebene, definiert, die quer zur Längsachse der Dichtung 336 verläuft, in einer Draufsicht kreisringförmig ausgebildet. Wellenberge 394 des ersten Rands 360 und Wellenberge 396 des zweiten Rands 364 erheben sich über die Öffnungsebene, Wellentäler 398 des ersten Rands 360 und Wellentäler 400 des zweiten Rands 364 liegen unterhalb der Öffnungsebene. Die in knickfreien Falten gelegte oder geraffte Wand 356 definiert somit ebenfalls sich in radialer Richtung von der Längsachse 26 weg erstreckende Faltenlinien 380. Die Dichtung 336 ist mit einer Dichtlippe 304 versehen, die in Form eines zylindrischen Rings aus einem elastischen Material ausgebildet ist. Eine Höhe der Dichtlippe 304 in axialer Richtung ist etwas größer als ein Abstand zwischen den Wellenbergen 394 und den Wellentälern 396 des ersten Rands 360.

Nachfolgend wird das Funktionsprinzip der Dichtungen 236 und 336 näher erläutert.

Durch die Öffnungen 288 und 388 können insbesondere Instrumente mit zylindrischem Schaft eingeführt werden. Ist ein Außendurchmesser eines Instruments größer als der Innendurchmesser der Öffnungen 288 und 388 der Dichtungen 236 und 336 in einer Grundstellung, in der die Öffnungen 288 und 388 ihren kleinsten Innendurchmesser aufweisen, so wird eine Abdichtung des Instrumentenschafts dadurch erreicht, dass die Faltung der Wände 256 und 356 geglättet wird, um die Öffnungen 288 und 388 im Innendurchmesser zu erweitern. Hierfür ist keine Dehnung der Wand 256 beziehungsweise 356 erforderlich. Wie bei den Ausführungsbeispielen in den Figuren 7 und 8 können jedoch Dichtlippen 204 und 304 vorgesehen sein, die Abdichtungseigenschaften der Dichtungen 236 und 336 sogar noch verbessern können, je nachdem, welche Art von Instrument mit den Dichtungen 236 und 336 abgedichtet werden soll.

Ferner können die Dichtungen 236 und 336 aus demselben Material oder denselben Materialien hergestellt sein, wie die Dichtungen 36 und 136. Selbstverständlich können auch die Dichtungen 236 und 336 mit einer reibungsmindernden Beschichtung, beispielsweise einer Gleitschicht aus Polytetrafluorethylen (PTFE), beschichtet sein. Die Dichtlippe 304 kann an den ersten Rand 360 angespritzt oder angeklebt sein. Ferner wäre es auch möglich, die Dichtlippe 304 mit dem ersten Rand 260 zu verschweißen.

In nicht näher dargestellter Weise können die Dichtungen 236 und 336 mit ihren zweiten Rändern 264 und 364 an einer entsprechenden Aufnahme eines Trokars festgelegt werden, um ein chirurgisches Abdichtungssystem auszubilden.

Allen oben beschriebenen Ausführungsbeispielen erfindungsgemäßer Dichtungen ist die Eigenschaft gemein, dass eine Öffnung durch Auffalten oder Verringern einer Faltung einer Wand der Dichtung erreicht wird, wobei trotzdem ein vollständiges Anliegen eines ersten Rands der Dichtung am Instrumentenschaft sichergestellt ist.

## Patentansprüche

1. Chirurgisches Dichtelement (52, 54; 136; 236; 336), insbesondere für einen Trokar (12) und/oder zum Abdichten von Schäften (102) langgestreckter chirurgischer Instrumente beim Einführen in einen menschlichen oder tierischen Körper, wobei das Dichtelement eine Längsachse (26) definiert, mit einer im Durchmesser veränderlichen und quer oder im Wesentlichen quer zur Längsachse (26) orientierten Öffnung (88; 188; 288; 388) versehen ist, durch die ein Instrument eingeführt werden kann, und eine ringförmig geschlossene, flexible Wand (56, 58; 156; 256; 356) umfasst, wobei die Wand (56, 58; 156; 256; 356) einen ersten (60, 62; 160; 260; 360) und einen zweiten (64, 66; 164; 264; 364), jeweils in sich geschlossenen Rand aufweist und wobei der erste Rand (60, 62; 160; 260; 360) die Öffnung (88; 188; 288; 388) begrenzt, **dadurch gekennzeichnet, dass** die Wand (56, 58; 156; 256; 356) wellenförmig faltbar ist und in einer Dichtstellung derart wellenförmig ohne Knicke mit in Richtung auf den ersten Rand (60, 62; 160; 260; 360) hin verlaufenden Faltlinien (80, 82; 180; 280; 380) gefaltet ist, dass der erste Rand (60, 62; 160; 260; 360) eine Wellenlinie (84, 86; 184; 284; 384) definiert, welche vollständig auf einer Zylinderfläche liegt.

2. Chirurgisches Dichtelement nach Anspruch 1, **dadurch gekennzeichnet, dass** die Zylinderfläche konzentrisch zur Längsachse (26) orientiert ist.

3. Chirurgisches Dichtelement nach einem der voranstehenden Ansprüche, **dadurch gekennzeichnet, dass** das Dichtelement (52, 54; 136; 236; 336) in einer Grundstellung eine Dichtungsstellung einnimmt, in der die Öffnung (88; 188; 288; 388) einen minimalen Durchmesser aufweist.

4. Chirurgisches Dichtelement nach einem der voranstehenden Ansprüche, **dadurch gekennzeichnet, dass** die Wellenlinie (84, 86; 184; 284; 384) oberhalb einer senkrecht zur Längsachse (26) verlaufenden Öffnungsebene (92; 192) der Öffnung (88; 188; 288; 388) Wellenberge (94; 194; 294; 394) und unterhalb der Öffnungsebene (92; 192) Wellentäler (98; 198; 298; 398) aufweist.

5. Chirurgisches Dichtelement nach einem der voranstehenden Ansprüche, **dadurch gekennzeichnet, dass** die Wand (56, 58; 156; 256; 356) in einer Vielzahl von Dichtstellungen mit jeweils unterschiedlichen Durchmessern der Öffnung (88; 188; 288; 388) in Richtung auf den ersten Rand (60, 62; 160; 260; 360) hin derart wellenförmig ohne Knicke gefaltet ist, dass der erste Rand (60, 62; 160; 260; 360) jeweils eine Wellenlinie (84, 86; 184; 284; 384) definiert, welche vollständig auf einer Zylinderfläche liegt.

6. Chirurgisches Dichtelement nach einem der voranstehenden Ansprüche, **dadurch gekennzeichnet, dass** der erste Rand (60, 62; 160; 260; 360) in Form einer Dichtlippe (204; 304) ausgebildet ist oder eine Dichtlippe (204; 304) trägt.

7. Chirurgisches Dichtelement nach Anspruch 6, **dadurch gekennzeichnet, dass** die Dichtlippe (204; 304) in Form eines Dichtrings (204; 304) oder in Form eines im Querschnitt kreisförmigen oder im Wesentlichen kreisförmigen Wulstes (204; 304) ausgebildet und vorzugsweise aus einem elastischen dehnfähigen Material hergestellt ist.

8. Chirurgisches Dichtelement nach einem der voranstehenden Ansprüche, **dadurch gekennzeichnet, dass** die Wand (56, 58; 156; 256; 356) aus einem inelastischen oder im Wesentlichen inelastischen Material hergestellt ist.

9. Chirurgisches Dichtelement nach einem der voranstehenden Ansprüche, **dadurch gekennzeichnet, dass** das Dichtelement (52, 54; 136; 236; 336) einstückig ausgebildet ist.

10. Chirurgisches Dichtelement nach einem der voranstehenden Ansprüche, **dadurch gekennzeichnet, dass** das Dichtelement (52, 54; 136; 236; 336) und/oder die Dichtlippe (204; 304) aus mindestens einem Kunststoff hergestellt ist.

11. Chirurgisches Dichtelement nach einem der voranstehenden Ansprüche, **dadurch gekennzeichnet, dass** eine unflexible Halterung (38) vorgesehen ist und dass der zweite Rand (64, 66; 164) in oder an der unflexiblen Halterung (38) gehalten ist.

12. Chirurgisches Dichtelement nach einem der voranstehenden Ansprüche, **dadurch gekennzeichnet, dass** das Dichtelement (52, 54; 136; 236; 336) mindestens teilweise mit einer reibungsmindernden Beschichtung versehen ist.

13. Chirurgisches Dichtelement nach einem der voranstehenden Ansprüche, **dadurch gekennzeichnet, dass** im Bereich des zweiten Randes (64, 66; 164; 264; 364) ein radial oder im Wesentlichen radial von der Längsachse (26) weg weisender Flansch ausgebildet, angeordnet oder angeformt ist.

14. Chirurgische Dichtung (36), insbesondere für einen Trokar (12) und/oder zum Abdichten von Schäften (102) langgestreckter chirurgischer Instrumente beim Einführen in einen menschlichen oder tierischen Körper, wobei die Dichtung ein erstes Dichtelement (52) umfasst, welches eine Längsachse (26) definiert, mit einer im Durchmesser veränderlichen und quer oder im Wesentlichen quer zur Längsachse (26) orientierten Öffnung (88) versehen ist, durch die ein Instrument eingeführt werden kann, und eine erste ringförmig geschlossene, flexible Wand (56) umfasst, wobei die erste Wand (56) einen ersten und einen zweiten, jeweils in sich geschlossenen Rand (60, 64) aufweist und wobei der erste Rand (60) die Öffnung (88) begrenzt, **dadurch gekennzeichnet, dass** die erste Wand (56) wellenförmig faltbar ist und in einer Dichtstellung derart wellenförmig ohne Knicke mit in Richtung auf den ersten Rand (60) hin verlaufenden Faltlinien (80) gefaltet ist, dass der erste Rand (60) eine Wellenlinie (84) definiert, welche vollständig auf einer Zylinderfläche liegt, dass ein zweites, identisch oder im Wesentlichen identisch mit dem ersten Dichtelement (52) ausgebildetes Dichtelement (54) mit einer zweiten Wand (58), die auch einen ersten und zweiten Rand (62, 66) aufweist, vorgesehen ist und dass der erste Rand (60) des ersten Dichtelements (52) mit dem ersten Rand (62) des zweiten Dichtelements (54) verbunden ist, so dass in der ersten Dichtstellung ein Wellenberg (94) einer durch den ersten Rand (60) des ersten Dichtelements (52) definierten ersten Wellenlinie (84) in ein Wellental (100) einer durch den ersten Rand (62) des zweiten Dichtelements (54) definierten Wellenlinie (86) eingreift.

15. Chirurgische Dichtung nach Anspruch 14, **dadurch gekennzeichnet, dass** das erste und/oder das zweite Dichtelement (52, 54) Dichtelemente (52, 54) nach einem der Ansprüche 2 bis 13 sind.

16. Chirurgisches Abdichtungssystem (10) zum Einführen chirurgischer Instrumente in einen menschlichen oder tierischen Körper, vorzugsweise längs einer vom Abdichtungssystem (10) definierten Längsachse (26), umfassend einen eine Einführöffnung (44) aufweisenden Trokar (12) und ein die Einführöffnung (44) mindestens teilweise verschließendes, eine Öffnung (88; 188; 288; 388) aufweisendes chirurgisches Dichtelement (52, 54; 136; 236; 336) zum Abdichten der Einführöffnung (44) beim Einführen eines Instruments in die Öffnung (88; 188; 288; 388), **dadurch gekennzeichnet, dass** das Dichtelement (52, 54; 136; 236; 336) ein Dichtelement (52, 54) nach einem der Ansprüche 1 bis 11 oder eine chirurgische Dichtung (36; 136; 236; 336) nach einem der Ansprüche 14 oder 15 ist.

17. Chirurgisches Abdichtungssystem nach Anspruch 16, **dadurch gekennzeichnet, dass** das Dichtelement (52, 54; 136; 236; 336) mit dem Trokar (12) lösbar verbindbar ist.

## Claims

1. Surgical sealing element (52, 54; 136; 236; 336), in particular, for a trocar (12) and/or for sealing shafts (102) of elongated surgical instruments during insertion into a human or animal body, the sealing element defining a longitudinal axis (26), being provided with an opening (88; 188; 288; 388) variable in diameter and oriented transversely or essentially transversely to the longitudinal axis (26), an instrument being insertable through the opening, and comprising a flexible wall (56, 58; 156; 256; 356) closed annularly, the wall (56, 58; 156; 256; 356) having a first edge (60, 62; 160; 260; 360) and a second edge (64, 66; 164; 264; 364) each closed upon itself, and the first edge (60, 62; 160; 260; 360) limiting the opening (88; 188; 288; 388), **characterized in that** the wall (56, 58; 156; 256; 356) is foldable in a wave-like manner and in a sealing position is folded in a wave-like manner without kinks with fold lines (80, 82; 180; 280; 380) extending in the direction towards the first edge (60, 62; 160; 260; 360) in such a manner that the first edge (60, 62; 160; 260; 360) defines a wave line (84, 86; 184; 284; 384) located entirely on a cylindrical surface.

2. Surgical sealing element as defined in claim 1, **characterized in that** the cylindrical surface is oriented concentrically to the longitudinal axis (26).

3. Surgical sealing element as defined in either one of the preceding claims, **characterized in that** in a basic position the sealing element (52, 54; 136; 236; 336) takes up a sealing position in which the opening (88; 188; 288; 388) has a minimum diameter.

4. Surgical sealing element as defined in any one of the preceding claims, **characterized in that** the wave line (84, 86; 184; 284; 384) has wave peaks (94; 194; 294; 394) above a plane of opening (92; 192) of the opening (88; 188; 288; 388) extending at right angles to the longitudinal axis (26) and wave troughs (98; 198; 298; 398) below the plane of opening (92; 192).

5. Surgical sealing element as defined in any one of the preceding claims, **characterized in that** in a plurality of sealing positions with respectively different diameters of the opening (88; 188; 288; 388) the wall (56, 58; 156; 256; 356) is folded in the direction of the first edge (60, 62; 160; 260; 360) in a wave-like manner without kinks in such a manner that the first edge (60, 62; 160; 260, 360) defines a respective wave line (84, 86; 184; 284; 384) located completely on a cylindrical surface.

6. Surgical sealing element as defined in any one of the preceding claims, **characterized in that** the first edge (60, 62; 160; 260; 360) is designed in the form of a sealing lip (204; 304) or bears a sealing lip (204; 304).

7. Surgical sealing element as defined in claim 6, **characterized in that** the sealing lip (204; 304) is designed in the form of a sealing ring (204; 304) or in the form of a bead (204; 304) circular or essentially circular in cross section and preferably is produced from an elastic stretchable material.

8. Surgical sealing element as defined in any one of the preceding claims, **characterized in that** the wall (56, 58; 156; 256; 356) is produced from a non-elastic or essentially non-elastic material.

9. Surgical sealing element as defined in any one of the preceding claims, **characterized in that** the sealing element (52, 54; 136; 236; 336) is designed in one piece.

10. Surgical sealing element as defined in any one of the preceding claims, **characterized in that** the sealing element (52, 54; 136; 236; 336) and/or the sealing lip (204; 304) is produced from at least one plastic material.

11. Surgical sealing element as defined in any one of the preceding claims, **characterized in that** a non-flexible holder (38) is provided, and **in that** the second edge (64, 66; 164) is held in or on the non-flexible holder (38).

12. Surgical sealing element as defined in any one of the preceding claims, **characterized in that** the sealing element (52, 54; 136; 236; 336) is provided at least partially with a coating reducing friction.

13. Surgical sealing element as defined in any one of the preceding claims, **characterized in that** a flange pointing radially or essentially radially away from the longitudinal axis (26) is formed, arranged or integrally formed in the area of the second edge (64, 66; 164; 264; 364).

14. Surgical seal (36), in particular, for a trocar (12) and/or for sealing shafts (102) of elongated surgical instruments during insertion into a human or animal body, the seal comprising a first sealing element (52) defining a longitudinal axis (26), being provided with an opening (88) variable in diameter and oriented transversely or essentially transversely to the longitudinal axis (26), an instrument being insertable through the opening, and comprising a first flexible wall (56) closed annularly, the first wall (56) having a first and a second edge (60, 64) each closed upon itself, and the first edge (60) limiting the opening (88), **characterized in that** the first wall (56) is foldable in a wave-like manner and in a sealing position is folded in a wave-like manner without kinks with fold lines (80) extending in the direction towards the first edge (60) in such a manner that the first edge (60) defines a wave line (84) located entirely on a cylindrical surface, **in that** a second sealing element (54) identical or essentially identical to the first sealing element (52) is provided and has a second wall (58) also with a first and second edge (62, 66), and **in that** the first edge (60) of the first sealing element (52) is connected to the first edge (62) of the second sealing element (54) such that in the first sealing position a wave peak (94) of a first wave line (84) defined by the first edge (60) of the first sealing element (52) engages in a wave trough (100) of a wave line (86) defined by the first edge (62) of the second sealing element (54).

15. Surgical seal as defined in claim 14, **characterized in that** the first and/or the second sealing elements (52, 54) are sealing elements (52, 54) as defined in any one of claims 2 to 13.

16. Surgical sealing system (10) for the insertion of surgical instruments into a human or animal body, preferably along a longitudinal axis (26) defined by the sealing system (10), comprising a trocar (12) having an insertion opening (44), and a surgical sealing element (52, 54; 136; 236; 336) closing the insertion opening (44) at least partially and having an opening (88; 188; 288; 388), the surgical sealing element serving to seal the insertion opening (44) during insertion of an instrument into the opening (88; 188; 288; 388), **characterized in that** the sealing element (52, 54; 136; 236; 336) is a sealing element (52, 54) as defined in any one of claims 1 to 11 or a surgical seal (36; 136; 236; 336) as defined in either claim 14 or 15.

17. Surgical sealing system as defined in claim 16, **characterized in that** the sealing element (52, 54; 136; 236; 336) is releasably connectable to the trocar (12).

## Revendications

1. Elément d'étanchéité chirurgical (52, 54; 136; 236; 336), notamment pour un trocart (12) et/ou pour assurer l'étanchéité de corps allongés (102) d'instruments chirurgicaux allongés lors de l'introduction dans un corps humain ou animal, l'élément d'étanchéité définissant un axe longitudinal (26) et étant pourvu d'une ouverture (88; 188; 288; 388) de diamètre variable, orientée transversalement ou sensiblement transversalement à l'axe longitudinal (26), à travers laquelle peut être introduit un instrument, et l'élément d'étanchéité comprenant une paroi flexible (56, 58; 156; 256; 356) fermée selon une forme annulaire, la paroi (56, 58; 156; 256; 356) présentant un premier (60, 62; 160; 260; 360) et un deuxième bord (64, 66; 164; 264; 364) chacun fermé en soi, et le premier bord (60, 62; 160; 260; 360) délimitant l'ouverture (88; 188; 288; 388),
**caractérisé en ce que** la paroi (56, 58; 156; 256; 356) peut être pliée sous forme ondulée, et, dans une position d'étanchéité, est pliée sous forme ondulée, sans plis à angle vif, avec des lignes de pliage (80, 82; 180; 280; 380) s'étendant en direction du premier bord (60, 62; 160; 260; 360), d'une manière telle que le premier bord (60, 62; 160; 260; 360) définisse une ligne ondulée (84, 86; 184; 284; 384) qui se situe en totalité sur une surface cylindrique.

2. Elément d'étanchéité chirurgical selon la revendication 1, **caractérisé en ce que** la surface cylindrique est orientée de manière concentrique à l'axe longitudinal (26).

3. Elément d'étanchéité chirurgical selon l'une des revendications précédentes, **caractérisé en ce que** l'élément d'étanchéité (52, 54; 136; 236; 336) prend, dans une position de base ou position initiale, une position d'étanchéité pour laquelle l'ouverture (88; 188; 288; 388) présente un diamètre minimal.

4. Elément d'étanchéité chirurgical selon l'une des revendications précédentes, **caractérisé en ce que** la ligne ondulée (84, 86; 184; 284; 384) présente, au-dessus d'un plan d'ouverture (92; 192) de l'ouverture (88; 188; 288; 388), qui s'étend perpendiculairement à l'axe longitudinal (26), des sommets d'onde (94; 194; 294; 394), et des creux d'ondes (98; 198; 298; 398) en-dessous du plan d'ouverture (92; 192).

5. Elément d'étanchéité chirurgical selon l'une des revendications précédentes, **caractérisé en ce que** la paroi (56, 58; 156; 256; 356) est, dans un grand nombre de positions d'étanchéité avec des diamètres respectifs différents de l'ouverture (88; 188; 288; 388), pliée sous forme ondulée sans plis à angle vif, en direction du premier bord (60, 62; 160; 260; 360), d'une manière telle que le premier bord (60, 62; 160; 260; 360) définisse à chaque fois une ligne ondulée (84, 86; 184; 284; 384) qui se situe en totalité sur une surface cylindrique.

6. Elément d'étanchéité chirurgical selon l'une des revendications précédentes, **caractérisé en ce que** le premier bord (60, 62; 160; 260; 360) est réalisé sous la forme d'une lèvre d'étanchéité (204; 304), ou porte une lèvre d'étanchéité (204; 304).

7. Elément d'étanchéité chirurgical selon la revendication 6, **caractérisé en ce que** la lèvre d'étanchéité (204; 304) est réalisée sous la forme d'une bague d'étanchéité (204; 304) ou sous la forme d'un bourrelet (204; 304) de forme circulaire ou sensiblement de forme circulaire en section transversale, et est de préférence fabriquée en un matériau élastique pouvant s'allonger.

8. Elément d'étanchéité chirurgical selon l'une des revendications précédentes, **caractérisé en ce que** la paroi (56, 58; 156; 256; 356) est fabriquée en un matériau non élastique ou sensiblement non élastique.

9. Elément d'étanchéité chirurgical selon l'une des revendications précédentes, **caractérisé en ce que** l'élément d'étanchéité (52, 54; 136; 236; 336) est réalisé d'un seul tenant.

10. Elément d'étanchéité chirurgical selon l'une des revendications précédentes, **caractérisé en ce que** l'élément d'étanchéité (52, 54; 136; 236; 336) et/ou la lèvre d'étanchéité (204; 304) est ou sont fabriqués en au moins une matière plastique.

11. Elément d'étanchéité chirurgical selon l'une des revendications précédentes, **caractérisé en ce qu'**il est prévu un support (38) non flexible, et **en ce que** le deuxième bord (64, 66; 164) est maintenu dans ou sur le support (38) non flexible.

12. Elément d'étanchéité chirurgical selon l'une des revendications précédentes, **caractérisé en ce que** l'élément d'étanchéité (52, 54; 136; 236; 336) est pourvu au moins en partie, d'un revêtement diminuant le frottement.

13. Elément d'étanchéité chirurgical selon l'une des revendications précédentes, **caractérisé en ce que** dans la zone du deuxième bord (64, 66; 164; 264; 364) est réalisé, agencé ou formé par moulage un flasque s'éloignant radialement ou sensiblement de manière radiale de l'axe longitudinal (26).

14. Joint d'étanchéité chirurgical (36), notamment pour un trocart (12) et/ou pour assurer l'étanchéité de corps allongés (102) d'instruments chirurgicaux allongés lors de l'introduction dans un corps humain ou animal, le joint d'étanchéité comprenant un premier élément d'étanchéité (52), qui définit un axe longitudinal (26), et étant pourvu d'une ouverture (88) de diamètre variable, orientée transversalement ou sensiblement transversalement à l'axe longitudinal (26), à travers laquelle peut être introduit un instrument, et l'élément d'étanchéité comprenant une première paroi flexible (56) fermée selon une forme annulaire, la première paroi (56) présentant un premier et un deuxième bord (60, 64) chacun fermé en soi, et le premier bord (60) délimitant l'ouverture (88),
**caractérisé en ce que** la première paroi (56) peut être pliée sous forme ondulée, et, dans une position d'étanchéité, est pliée sous forme ondulée, sans plis à angle vif, avec des lignes de pliage (80) s'étendant en direction du premier bord (60), d'une manière telle que le premier bord (60) définisse une ligne ondulée (84) qui se situe en totalité sur une surface cylindrique, **en ce qu'**il est prévu un deuxième élément d'étanchéité (54) d'une configuration identique ou sensiblement identique au premier élément d'étanchéité (52) et comprenant une deuxième paroi (58) qui présente également un premier et un deuxième bord (62, 66), et **en ce que** le premier bord (60) du premier élément d'étanchéité (52) est relié au premier bord (62) du deuxième élément d'étanchéité (54) de manière à ce que dans la première position d'étanchéité, un sommet d'onde (94) d'une première ligne ondulée (84) définie par le premier bord (60) du premier élément d'étanchéité (52), s'engage dans un creux d'onde (100) d'une ligne ondulée (86) définie par le premier bord (62) du deuxième élément d'étanchéité (54).

15. Joint d'étanchéité chirurgical selon la revendication 14, **caractérisé en ce que** le premier et/ou le deuxième élément d'étanchéité (52, 54) sont des éléments d'étanchéité (52, 54) selon l'une des revendications 2 à 13.

16. Système d'étanchéité chirurgical (10) destiné à l'introduction d'instruments chirurgicaux dans un corps humain ou animal, de préférence le long d'un axe longitudinal (26) défini par le système d'étanchéité (10), comprenant un trocart (12) qui présente une ouverture d'introduction (44), et un élément d'étanchéité chirurgical (52, 54; 136; 236; 336) qui ferme au moins partiellement l'ouverture d'introduction (44) et présente une ouverture (88; 188; 288; 388), pour assurer l'étanchéité de l'ouverture d'introduction (44) lors de l'introduction de l'instrument dans l'ouverture (88; 188; 288; 388), **caractérisé en ce que** l'élément d'étanchéité (52, 54; 136; 236; 336) est un élément d'étanchéité (52, 54) selon l'une des revendications 1 à 11, ou un joint d'étanchéité chirurgical (36; 136; 236; 336) selon l'une des revendications 14 ou 15.

17. Système d'étanchéité chirurgical selon la revendication 16, **caractérisé en ce que** l'élément d'étanchéité (52, 54; 136; 236; 336) peut être relié de manière amovible avec le trocart (12).
